# NEW EUROPEAN PATENT SPECIFICATION

(11) **EP 2 302 380 B2**
(45) Date of publication and mention of the opposition decision: **11.11.2015**
(45) Mention of the grant of the patent: 21.11.2012
(21) Application number: 09012276.3
(22) Date of filing: 28.09.2009
(51) Int. Cl.: G01N 33/68

(54) **Method for preclinical testing of immunomodulatory drugs**
Verfahren zum vorklinischen Testen von immunomodulatorischen Arzneimitteln
Procédé de test préclinique de médicaments immunomodulateurs

(43) Date of publication of application: 30.03.2011
(73) Proprietor: TheraMab GmbH, 97076 Würzburg (DE)
(72) Inventor: Hünig, Thomas, 97286 Winterhausen (DE)
(74) Representative: Vossius & Partner Patentanwälte Rechtsanwälte mbB

(56) References cited:
- EP-A1- 1 944 359
- WO-A1-2008/081198
- US-A1- 2006 286 104
- STEBBINGS R ET AL: "Safety of biologics, lessons learnt from TGN1412" CURRENT OPINION IN BIOTECHNOLOGY, LONDON, GB, vol. 20, no. 6, 1 December 2009 (2009-12-01), pages 673-677, XP026778877 ISSN: 0958-1669 [retrieved on 2009-11-04]

## Description

### Field of the invention

The invention relates to a method wherein a known or prospective immunomodulatory drug is tested, comprising the step of contacting an in-vitro peripheral blood mononuclear cell (PBMC) culture to the immunomodulatcry drug and observing the PBMC culture for release of at least one cytokine from the PBMC or for cell proliferation upon contact with the immunomodulatory drug. The invention further relates to a method of testing cytokine storm attenuating drugs in-vitro. The invention finally relates to a method.

### State of the art and background of the invention

Immunotherapeutic drugs which modulate the activity of lymphocytes are preclinically evaluated in two systems: Animal models, usually rodents, and, if available, primate; and cultures of human peripheral blood mononuclear cells (PBMC).

PBMC are routinely used because, first, they contain all subsets of lymphocytes as well as monocytes, and, second, they are readily available from venous blood drawn from healthy donors or from patients. In vitro stimulation of these PBMC is considered a useful indicator of the activities of an immunomodulatory drug to be expected in vivo.

Certain T-cell activating agents, in particular monoclonal antibodies (mAb) addressing the T-cell antigen receptor (TCR) such as OKT3, the first mAb used in the clinic for immunosuppression, induce the systemic release of proinflammatory cytokines (Abramowicz et al., 1989). The most dangerous of these are TNF, interferon-gamma (IFN-gamma) and IL-2. In patients receiving mAb therapies, control of such a cytokine release syndrome or "cytokine storm" is routinely achieved by high dose corticosteroid treatment.

TGN1412 is a humanized monoclonal antibody (mAb) of the IgG4 subclass specific for the costimulatory molecule CD28 expressed by human T-cells. It is called a "CD28 superagonist" (CD28SA) because unlike the classic CD28-specific mAb, it can activate T-lymphocytes without simultaneous engagement of the T-cell antigen receptor (TCR) (Hunig, 2007). TGN1412 was developed by the now defunct TeGenero AG, Würzburg.

During a first-in-man trial conducted by the independent Parexel Clinical Trial Unit at Northwick Park Hospital, London, on March 13, 2006, intravenous application of 100µ/kg body weight of TGN1412 to healthy human volunteers led to a life-threatening cytokine release syndrome that was only controlled after transfer of the volunteers to the hospital's intensive care unit (Suntharalingam et al., 2006).

The preclinical work presented by the sponsor, TeGenero AG, showed no evidence for such a "cytokine storm" in an analogous rat model using a rat-CD28-specific superagonist, and in cynomolgus monkeys receiving TGN1412 itself at up to 50 fold higher doses than the human volunteers (Duff, 2006). Furthermore, addition of TGN1412 to cultures of human PBMC also did not result in cytokine release. All key monkey and PBMC culture experiments were repeated by the British National Institute for Biological Standards and Control (NIBSC) acting on behalf of the government's Expert Scientific Group on Phase One Clinical Trials, and confirmed the innocuous behaviour of TGN1412 in these systems (Duff, 2006). Three years after the failed TNG1412 trial, it still has not been clarified why this in vitro assay did not warn against the cytokine storm experienced by the human volunteers.

The failure of rodents and cynomolgous monkeys to release toxic systemic cytokines after injection of CD28SA is obviously due to interspecies differences in the reactivity of the intact immune system to such agents, and specific suggestions for such differences have been made. (Gogishvili et al., 2009; Nguyen et al., 2006; Schraven and Kalinke, 2008).

These findings indicate that because of species-specific reaction patterns, even primate animal models are not always safe predictors of human reactivity to novel drugs. It should be noted that a human being has roughly 10¹² T-lymphocytes, and that less than one percent of these are circulating in the blood at any given moment. Therefore, the failure of cultured PBMC to respond to TGN1412 is either due to a functional defect in these cells as compared to those residing in lymphoid tissues (which obviously responded with cytokine release in the volunteers), or to the requirement of a cell type present in lymphoid organs but not in blood for TGN1412-mediated activation of T-lymphocytes.

Reproduction of the cytokine storm observed in the human volunteers of the London TGN1412 trial in cell culture is, therefore, urgently needed to understand its mechanism and to test its sensitivity to pharmacological suppression.

From a broader point of view, the failure of known human PBMC cultures to respond to soluble TGN1412 with cytokine release indicates that this system does not respond to all lymphocyte-activating agents in the same fashion as does the intact human immune system inside the body. Correction of this defect may not only allow a detailed analysis of the effects of human CD28 superagonists (SA) such as TGN1412, but may also reveal the reactivity of other, seemingly innocuous drugs during preclinical development.

### Technical problem of the invention

The technical problem of the invention is, accordingly, to provide improved means for in-vitro testing of immunomodulatory drugs, in particular CD28SA, with respect to potential cytokine storms. A further object of the invention is to provide means for testing drugs suitable for attenuating cytokine storms.

Principles of the invention and preferred embodiments.

For solving the first-mentioned technical problem the invention teaches a method for testing a prospective or known immunomodulatory drug for T-cell activation as defined by the claims, comprising the step of contacting in-vitro a peripheral blood mononuclear cell (PBMC) culture with a predetermined amount of the prospective or known immunomodulatory drug and observing the PBMC culture for release of at least one cytokine from the PBMCs upon contact with the prospective or known immunomodulatory drug, wherein the cell density of a PBMC preculture is adjusted such that cell-cell contact of the PBMC is enabled and wherein the PBMC preculture is cultured for at least 24h. The term "precultured" means that the PBMC culture is cultured in absence of immunomodulatory drugs and prior to contact with such drugs to be tested. The term "observe" comprises the qualitative, half-quantitative and quantitative measurement of concentrations of the at least one cytokine or of proliferation with known methods of the art.

The invention is based on the surprising finding that a PBMC culture, which is prepared by standard methods, but further precultured for a predetermined period of time prior to contact with the immunomodulatory drug suddenly shows sensitivity with respect to cytokine release triggered by contact with immunomodulatory drugs, which do not trigger cytokine release in absence of the preculture process. The invention is further based on the finding that this preculture effect is promoted by cell-cell contacts of the PBMC for a predetermined period of time. Said with other words, the PBMC culture should not be freshly prepared when the immunomodulatory drug is added.

Therefore, the invention is useful to predict the reactivity of individuals to immunomodulatory drugs, like TGN1412, and, as will be explained later in more detail, the ability of immunosuppressant drugs such as corticosteroids to control unwanted reactions. It is also useful to further understand the mode of action of CD28SA. Moreover, the invention is useful in screening prospective immunomodulatory drugs for their T-cell activating potential, including cytokine release. Since the PBMC recovered after high-density preculturing most likely reflect the status of T-cell reactivity found in the lymphoid organs, they should, in combination with an activating agent, also be employed to test immunosuppressant drugs because circulating T-cells may be more easily suppressed due to their "inactive" status, resulting in misleading results on the efficacy of such drugs.

In the invention, the preculture step is carried out by storing the PBMC culture for at least 24 h, preferably at least 36h, more preferably at least 45 h, at 35 °C to 40 °C, preferably at 36 °C to 38 °C, in absence of immunomodulatory drugs, and prior to the contact with the immunomodulatory drug to be tested.

In the invention the cell density of the PBMC culture during and/or after the preculture step is at least 2*10⁶/ml, preferably at least 5*10⁶/ml, more preferably at least 10⁷/ml. In terms of cell density at the surface of the tissue culture vessel, it should be at least 4 * 10⁵/cm², preferably at least 10⁶/cm², most preferably at least 2 * 10⁶/cm². The values provided apply directly to vessels consisting of flat wells. In round wells or conical wells the overall density will differ in that the cell density is high at the bottom of the well and low in upper parts of the well. Accordingly the above given cell densities per unit of volume shall refer to partial volumes in vessels of any kind as well, i.e. the given densities shall be provided in a partial volume of e.g. at least 10µl of the total preculture volume present. Also any other method of achieving cell densities of viable cells, as claimed, shall be encompassed in the invention. Preferably, the minimum number of cells being in cell-cell-contact is at least 50,000.

In a preferred embodiment the immunomodulatory drug is an immunostimulating drug, like an antibody, preferably a monoclonal antibody. Specifically, the monoclonal antibody can be a human CD28 specific superagonistic monoclonal antibody.

In another embodiment of the invention the cytokine observed is selected from the group consisting of TNF, IFN-gamma, IL-1-beta, IL-1, IL-2, IL-3, IL-4, IL-5, IL-6, IL-7, IL-8, IL-9, IL-10, IL12p70, IL-13, IL-14, IL-15, IL-16, IL-17, IL-21, IL-35, LT, and combinations thereof. Alternatively proliferation of the cell can be observed, wherein proliferation is present, if the number of cells increases within a time unit by a predetermined amount. This amount can be chosen by the artisan by usual consideration.

In a further embodiment of particular relevance with respect to the second-mentioned object, the precultured PBMC culture is additionally contacted with a prospective drug for attenuating the release of at least one cytokine (immunosuppressant drug), at the same time as contacting of the PBMC culture with the known immunomodulatory, in particular stimulating, drug or subsequently after a predetermined period of time, or a predetermined period of time there before, wherein the cytokine release is further observed. The predetermined period of time can be in the range of 10 s to 12 h, preferably in the range of 10 s to 1 h.

The prospective drug for attenuating the cytokine release is preferably (but not exclusively) selected from the group consisting of corticosteroids, including, but not limited to dexamethasone or methylprednisolone.

In this embodiment it becomes possible to test in vitro, whether a particular drug intended for attenuation of a cytokine storm (immunosuppressant drug) is suitable, should such cytokine storm take place in in vivo experiments despite administration of the immunomodulatory drug at a concentration, at which a cytokine storm would not be expected. In particular, this variant of the invention allows creating a matching pair of immunomodulatory drug and attenuating drug and provides safe means for managing unexpected cytokine storms in in vivo experiments, in particular clinical trials with humans.

In the following the invention is explained in detail by examples and figures. The figures show:
- Figure 1:: Induction of cytokine release by the CD3-specific mAb OKT3 and the CD28 superagonist TGN1412 from human PBMC.
- Figure 2:: Consistent conversion from a TGN1412 nonreactive to a TGN1412-reactive state by 2 day preculture.
- Figure 3:: Acquisition of TGN1412-reactivity requires high cell density during preculture.
- Figure 4:: Acquisition of TGN1412-reactivity in high-density precultures requires two days of incubation.
- Figure 5:: Acquisition of TGN1412-reactivity in high-density cultures requires cell-cell contact.
- Figure 6:: Precultured, but not fresh PBMC respond to TGN1412 with proliferation.
- Figure 7:: CD45RO (memory) CD4 T-cells are the main source of proinflammatory cytokines released by both OKT3 and TGN1412.
- Figure 8:: Comparable kinetics of TNF release from high- density precultured PBMC induced by OKT3 and TGN1412.
- Figure 9:: Acquisition of TGN1412-reactivity is blocked by mAb to HLA antigens.
- Figure 10:: Comparable sensitivity of cytokine release induced by OKT3 and TGN1412 to corticosteroid- mediated suppression.

### Example 1: Comparative example

For the induction of cytokine release, the present invention as well as this comparative example uses the standard system of PBMC stimulation as it is employed by investigators all over the world to study the response of human PBMC to immune-modulating agents. This system employs freshly prepared PBMC, isolated from heparinized venous blood by centrifugation over a density gradient (Lymphocyte Separation Medium LSM 1077, PAA Laboratories, Pasching, Germany) following the manufacturer's instructions. Alternatively, a fresh leukocyte concentrate, obtained from leukocyte reduction system chambers (Caridian, Gambro BCT, Lakewood, CO, USA) as a byproduct in the preparation of platelet concentrates (Dietz et al., 2006) is used as starting material for Ficoll purification, with identical results. PBMC are cultured in 96-well tissue culture plates (Greiner bio-one, Frickenhausen, Germany), in which 2x10⁵ cells are stimulated in 0.2ml of enriched RPMI 1640 culture medium (GIBCO/Invitrogen, Long Island, NY, USA) supplemented with 10 % autologous serum or commercially available pooled AB serum (PAA Laboratories), with identical results.

In this tissue culture system, freshly isolated PBMC were stimulated with soluble TGN1412 provided by TheraMab GmbH, Würzburg. It was of the same GMP-quality batch used during the London trial (Suntharalingam et al., 2006). As a positive control for the induction of cytokine release, clinical-grade OKT3 ("Muromonomab", Janssen-Cilag, Neuss, Germany) was used, which is well known to trigger cytokine release both in vitro and in patients (Abramowicz et al., 1989). After 24 hours, a panel of cytokines including the major pro- and anti-inflammatory factors detected in plasma of the volunteers of the TGN1412 trial (Suntharalingam et al., 2006) were analyzed by cytokine-bead-array (CBA) technology (Becton Dickinson, Mountain View, CA, USA) following the manufacturer's instructions.

The concentration of both OKT3 and TGN1412 employed was 1 µg/ml, which is in the range of the estimated concentration achieved in the circulation of the volunteers during the London TGN1412 trial (Duff, 2006). Extensive titrations of both antibodies not displayed here showed that this concentration is within the optimum range for biological responses.

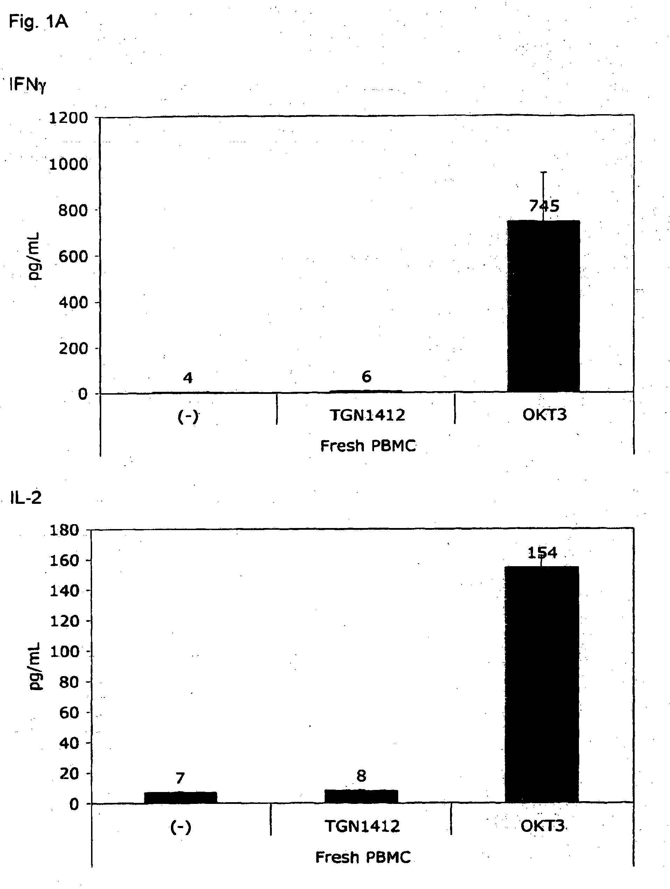

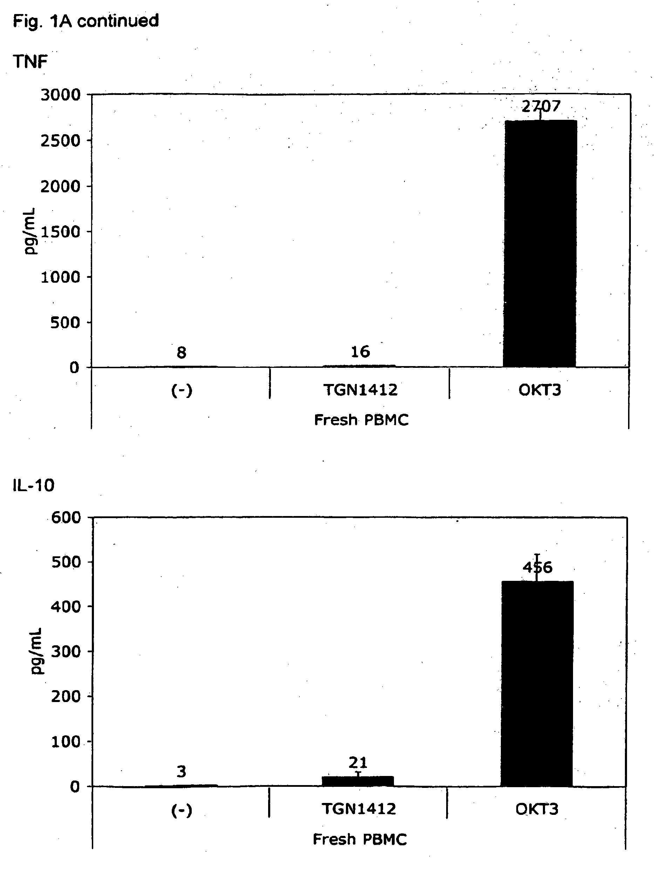

Figure 1A shows that soluble TGN1412 fails to induce cytokine release in fresh PBMC. In contrast, OKT3 is highly effective in inducing TNF, IFN-gamma, and IL-2, which are all known to contribute to the pathological manifestations of the cytokine release syndrome, as well as the anti-inflammatory cytokine IL-10. PBMC from a healthy donor were isolated by Ficoll density centrifugation and cultured in 0.2ml RPMI1640 medium supplemented with 10% AB serum for 24 hours at 10⁶/ml in 96 well flat bottom tissue culture plates. Cytokines in the supernatants were analyzed after 24 hours of incubation by cytokine bead array. Monoclonal antibodies were used at 1µg/ml final concentration. Mean values and standard deviations of triplicates are shown.

The failure of TGN1412 and the ability of OKT3 to induce release of these and other cytokines (not shown) in such standard PBMC cultures has been reproduced with over 10 individual donors, and is in agreement with the data submitted by TeGeneroAG and reproduced in the report of the Scientific Expert Group on the safety of Phase I clinical trials (Duff, 2006).

### Example 2: Response to TGN1412 after preculturing

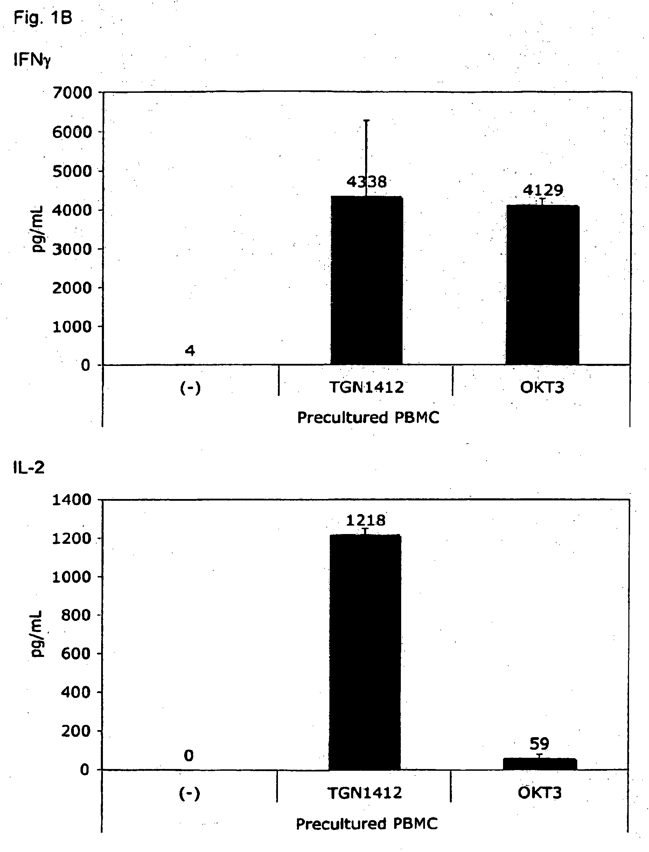

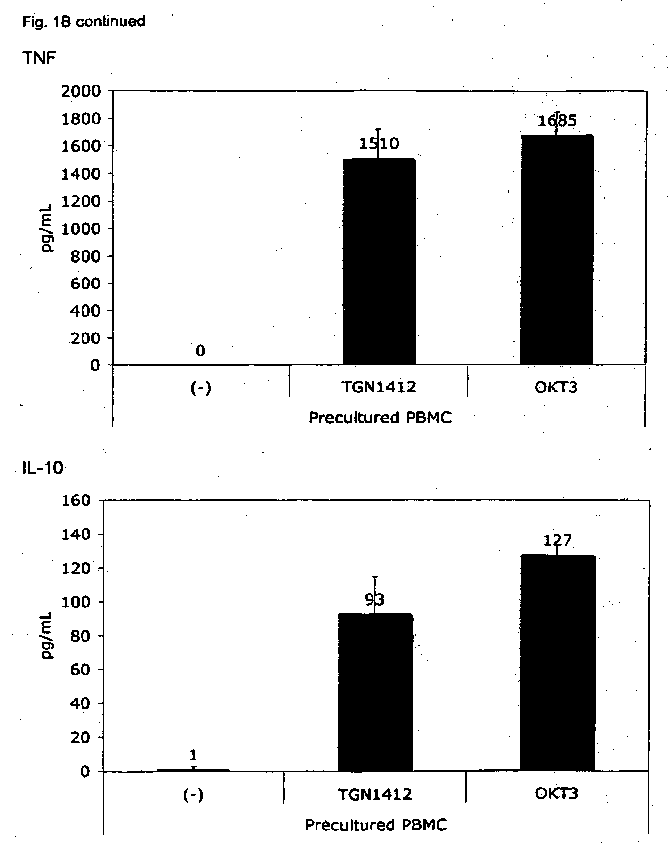

For the experiment in Fig 1B PBMC from a healthy donor were cultured in 1.5 ml medium for 2 days at 10⁷/ml in 24 well flat bottom tissue culture plates before being washed and readjusted to 10⁶/ml. With these cells, the same experiment was performed as described in Example 1.

Figure 1B shows that surprisingly, responsiveness to TGN1412 is restored by simply preculturing PBMC for 2 days without overt stimulation. When cells were prepared on December 4th 2008, the number of PBMC obtained exceeded that required for the current experiment, and surplus cells were stored in culture medium for two days at 37°C. When these cells were employed for exactly the same experiment as previously performed with fresh cells (Fig. 1A), something completely unexpected happened: TGN1412 now induced a cytokine release of comparable magnitude as OKT3. Fig. 1B provides an example of such an experiment.

The reproducibility and mechanistic basis were therefore further investigated.

### Example 3: Reproducibility of the key observation

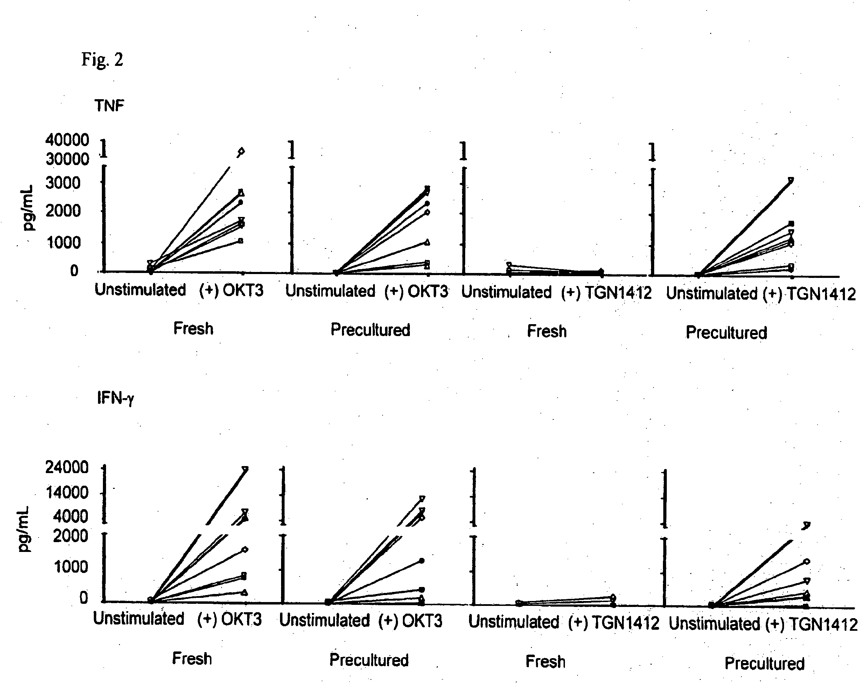

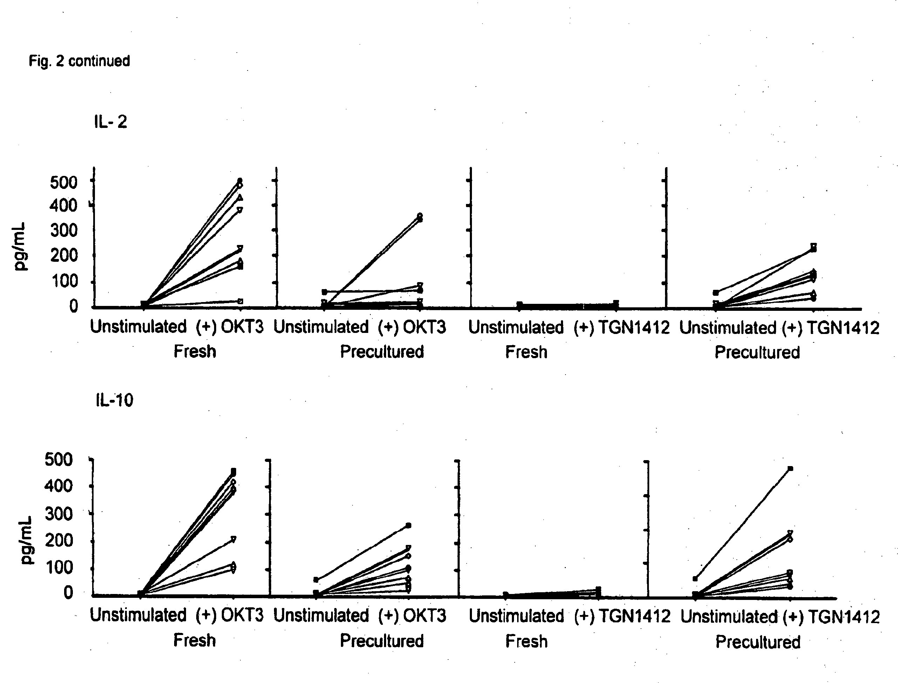

Figure 2 summarizes the effect of preculture on the reactivity to TGN1412 for 7 individual healthy donors. Data from 7 individual healthy donors are shown, each represented by a symbol. Conditions for antibody stimulation and for preculture were as in Figure 1. While donor-specific variations exist for both, OKT3 and TGN1412 responses, it is apparent that in all cases, fresh donor cells failed to respond to TGN1412 stimulation with cytokine release and that this refractory state was lost after 2 days of preculture. Donor-specific variations are expected, as is illustrated by the great differences in the magnitude of the cytokine storm experienced by the volunteers of the London TGN1412 trial (Suntharalingam et al., 2006).

### Example 4: Optimization of the new method.

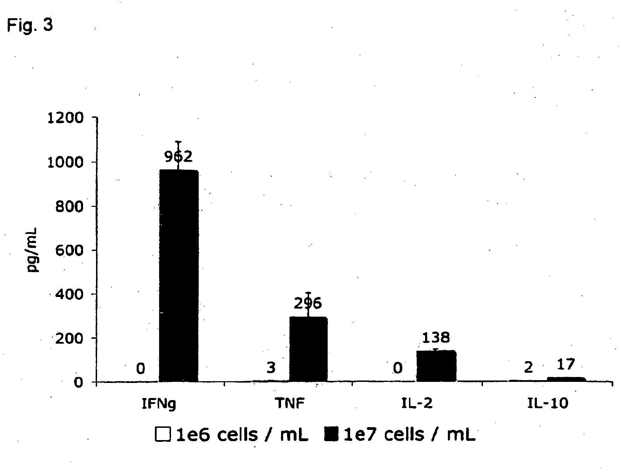

Figures 3 to 5 describe the parameters determining the acquisition of sensitivity to TGN1412 of peripheral blood T-cells.

For the experiment of Figure 3, PBMC were cultured at 10⁶/ml or at 10⁷/ml for 2 days before being stimulated by TGN1412 at 10⁶/ml as described in Figure 1.

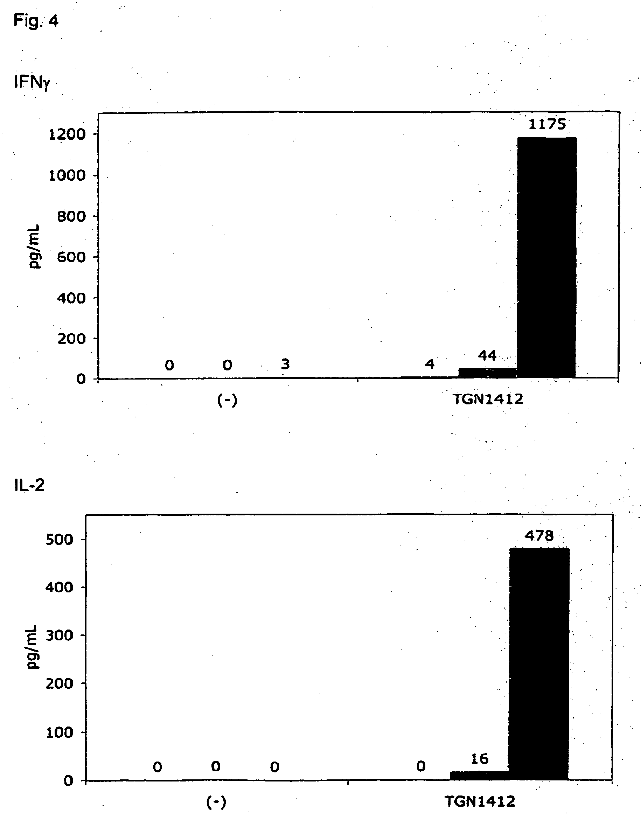

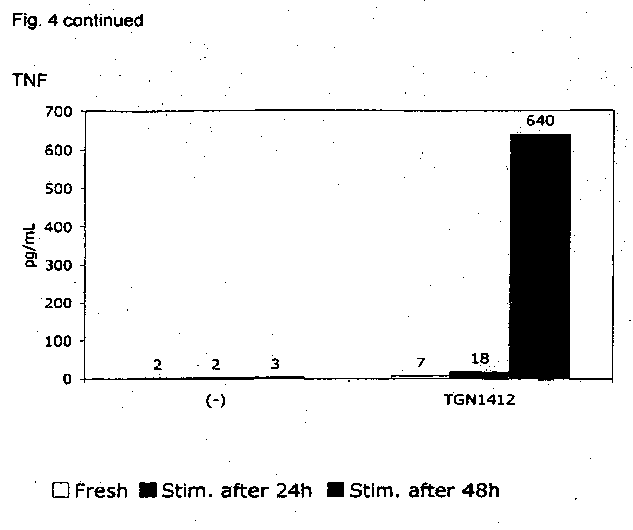

For the experiment of Figure 4, fresh PBMCs, (left bars of triplets) and PBMCs precultured at 10⁷/ml for 24 (middle bars of triplets) or 48 hours (right bars of triplets) were stimulated with TGN1412 for 24 hours under the conditions given in Figure 1.

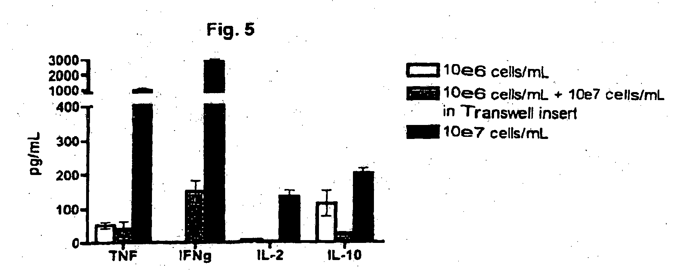

For the experiment of Figure 5, PBMC were cultured for two days at high density (10⁷/ml, right bars of triplets) or at low density (10⁶/ml, left and middle bars of triplets) in 1.5 ml transwell cultures containing an insert with a semipermeable membrane on which PBMC were cultured at high-density (10⁶/ml below membrane, 10⁷/ml above membrane, middle bars of triplets). Cells were restimulated and assayed as given in Figure 1.

As shown above, PBMC acquire sensitivity to TGN1412 by preculture in medium with 10% autologous or commercially available AB serum. We tested the role of the following parameters in the acquisition of TGN1412 sensitivity.

Cell density. In contrast to standard PBMC stimulation assays where cells are cultured at 10⁶/ml or 2x10⁵/cm² of the culture well, "parking" for 2 days was performed at a 10 fold higher density. Figure 3 shows that preculturing PBMC at high (10⁷/ml or 2x10⁶/cm²), but not at low (10⁶/ml or 2x10⁵/cm²) density induces reactivity to TGN1412 in the secondary culture.

Time. Figure 4 shows that full reactivity to TGN1412 (comparable to reactivity to OKT3) is achieved after 2 days of pre-culture. 1 day of preculture leads only to a modest increase of reactivity.

Requirement for cell-cell contact. The requirement for high cell density during preculture of PBMC for the acquisition of reactivity to TGN1412 can be due to the need for cell-cell contact and/or to the action of soluble factors which need to reach a certain concentration to promote maturation to the reactive state. Using a transwell system (Corning incorporated, Lowell, MA, USA), where cells cultured at high density are separated from those cultured at low density by an 8µm-pore membrane permitting the diffusion of soluble factors, it is shown in Figure 5 that cell-cell contact is required.

### Example 5: Further characteristics of the new method

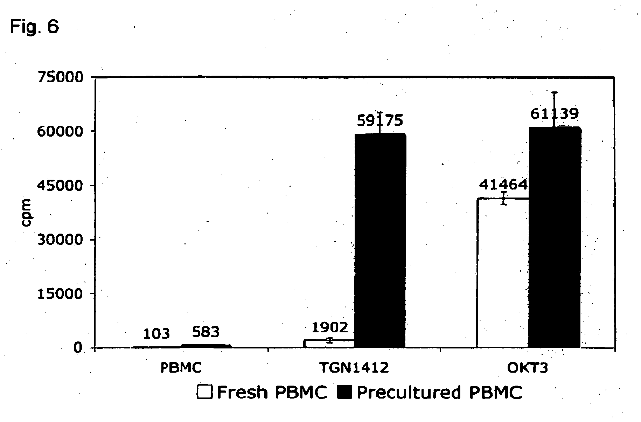

Figure 6 shows that precultured, but not freshly isolated PBMC proliferate in response to TGN1412. Fresh and precultured PBMC were prepared and cultured as described for Fig. 1A and B, respectively. On day 3, 1µCi of ³H-Thymidine was added, and cultures were harvested 16 hours later and processed for liquid scintillation counting. Besides cytokine release, polyclonal T-cell activation results in proliferation which can be measured as radioactivity incorporated as tritiated Thymidine. As is seen in Figure 2, OKT3 stimulated the proliferation of both, fresh and precultured PBMC, whereas TGN1412 was only able to induce proliferation in precultured PBMC. Thus, proliferation can also be used as a readout.

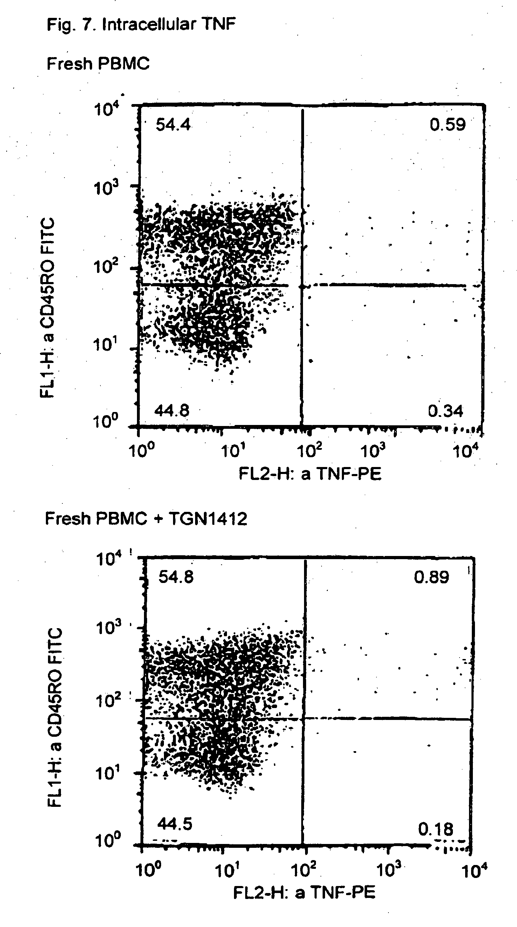

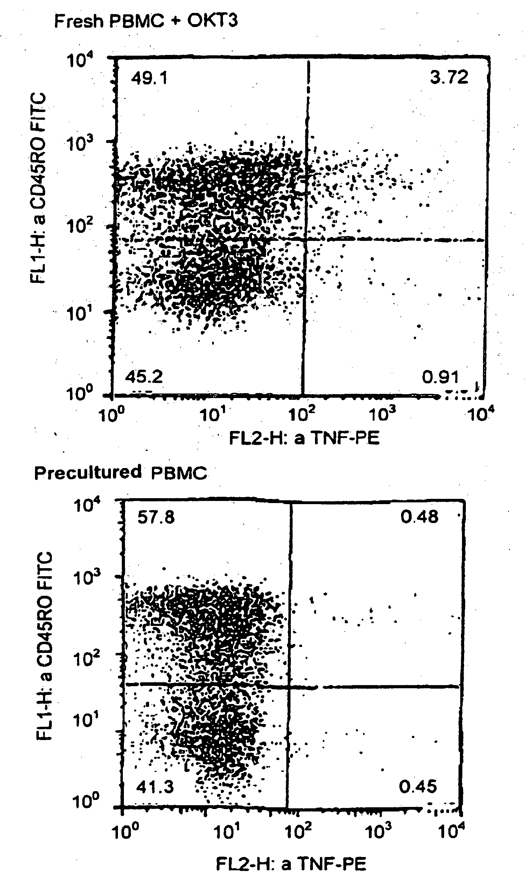

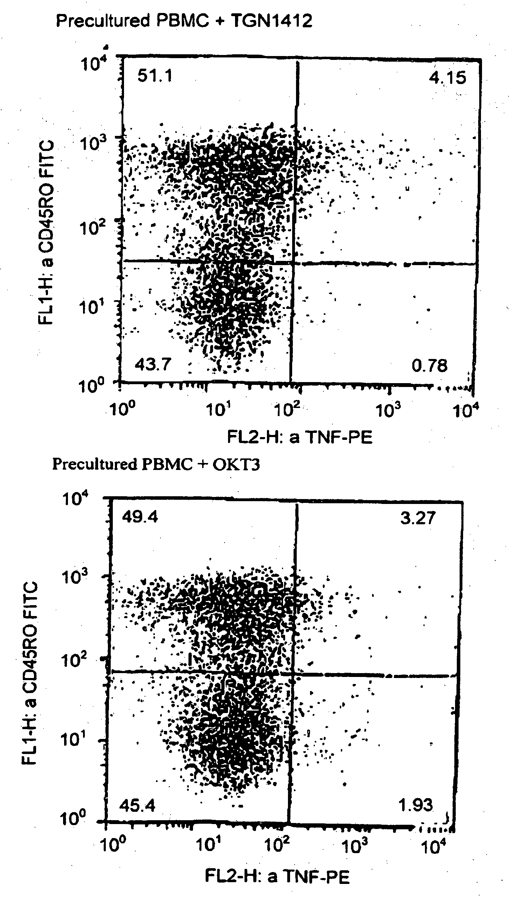

Figure 7 shows that in precultured PBMC, TGN1412 releases proinflammatory cytokines from CD4 memory cells. Fresh and 2 day high-density precultured PBMC were stimulated for 16 hours with 1 µg/ml of OKT3 or TGN1412. During the last 4 hours of culture, 5 µg/ml of brefeldin A was added to block cellular export of cytokines. After cell-surface staining with fluorochrome-conjugated mAb to CD4 and CD45RO (memory marker), cells were fixed, permeabilized and stained with mAb to TNF (all from BD Pharmingen, Mountain View, CA, USA). 15.000 live-gated events were acquired on a BD Calibur flow cytometer and data were analyzed using FlowJo software (Three Star Inc., Ashland, OR, USA). Data shown are gated on CD4 T-cells. The cytokine storm elicited by OKT3 in patients is a well-known phenomenon, and the accompanying information provided with this drug (Muromonomab, Janssen-CILAG) explicitly warns against this syndrome. To compare the cellular source of the two key pro-inflammatory cytokines TNF and IFN-gamma in response to OKT3 and TGN1412, they were retained within the cell by blockade of their transport through the Golgi apparatus using the drug brefeldin A (Sigma Aldrich, Steinheim, Germany), and revealed by intracellular staining of fixed and permeabilized cells with fluorescent TNF-specific mAb (Becton Dickinson, Mountain View, CA, USA). In parallel, the cell surface phenotype of the stimulated PBMC was determined. The main cytokine-producing T-cell subset, the CD4 T-cells, were thus identified and further subdivided into naive (CD45R0-) and memory (CD45RO+) subsets. Figure 7 shows that in both fresh and precultured PBMC, OKT3 triggers cytokine production predominantly in the CD4 memory cell subset. Moreover, TGN1412, while unable to trigger TNF production in fresh PBMC, efficiently does so in the same population of memory CD4 cells after high density preculture.

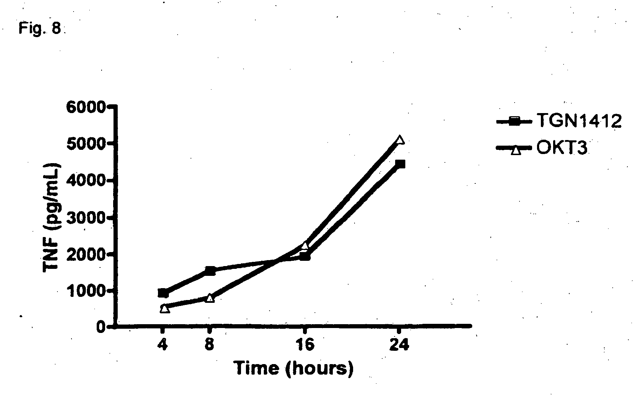

Figure 8 shows that TNF release from precultured PBMC follows the same kinetics when induced by either OKT3 or TGN1412. High-density precultured PBMC were prepared as in Fig. 1B and stimulated with 1µg/ml of OKT3 or TGN1412. Supernatants were harvested at the times indicated and TNF content was analysed as in Figure 1.

In vivo, the release of TNF, the most important pro-inflammatory cytokine of the "cytokine storm", follows the same kinetics when induced by either OKT3 or TGN1412 (Abramowicz et al., 1989; Suntharalingam et al., 2006). Therefore the kinetics of TNF-release in precultured PBMC between the two mAb were compared and found to be virtually identical.

### Example 6: Mechanistic basis for the induction of reactivity to TGN142 during high-density preculture: Self-MHC recognition

This Example provides functional information about the invention without being bound to theory. It has been suggested from work done in mice that recognition of molecules of the major histocompatibility complex (MHC; HLA in humans) by the antigen receptors (TCR) of T-cells in the lymphoid organs (lymph nodes, spleen, etc.) primes the TCR for enhanced signalling during antigen encounter later on (Stefanova et al., 2002). Since the inventor's laboratory had earlier shown that CD28 superagonist signalling acts through the amplification of weak TCR signals (Dennehy et al., 2007), it was hypothesized that it is the loss of this priming signal which makes circulating T-cells (which do not have cell-cell contact, unlike to the situation in the lymphoid organs) refractory to TGN1412 stimulation. This was tested by including blocking mAbs which react with all human HLA class I and class II molecules during the two days of preculture (mAbs 646-2.6 and Tü39, Becton-Dickinson) at 10µg/ml.

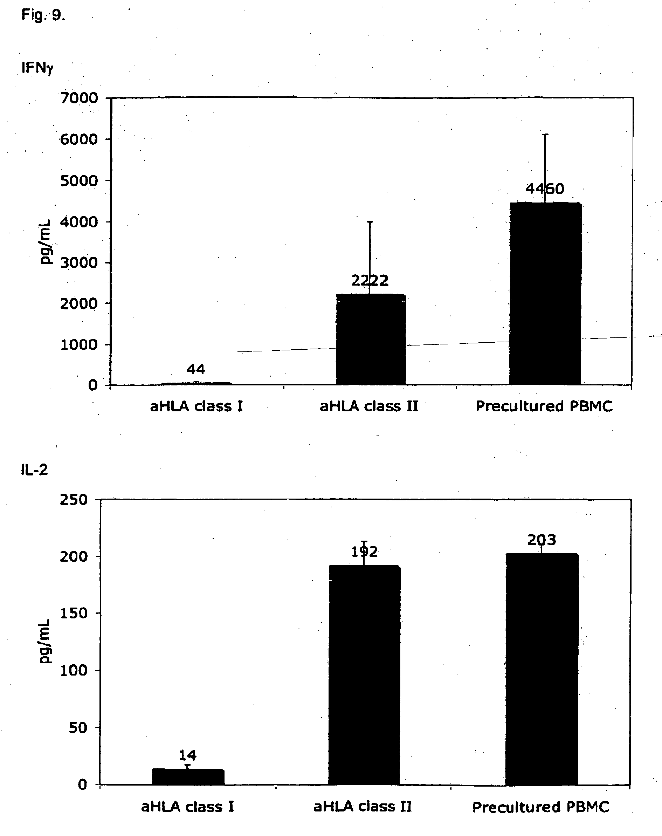

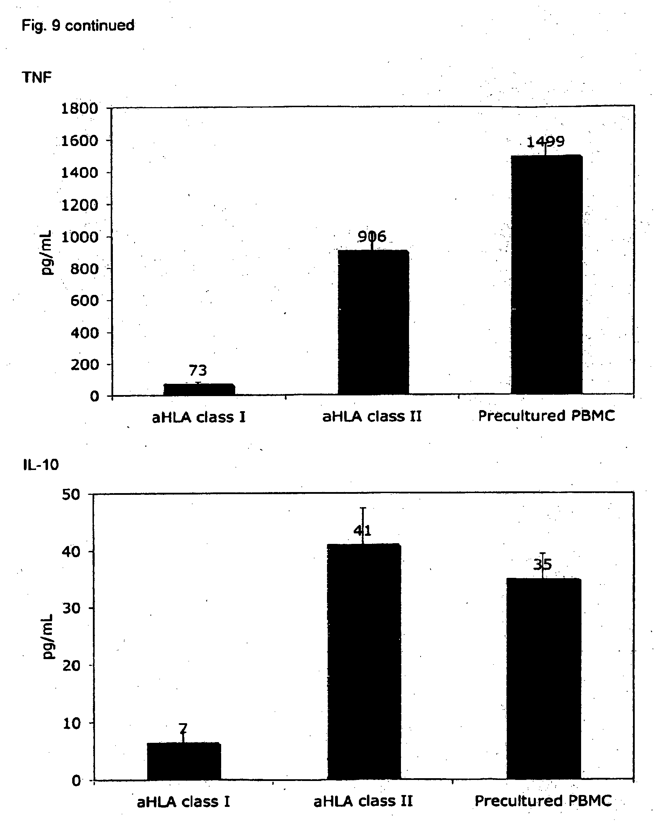

Figure 9 shows that HLA class I and, to a lesser extent HLA class II specific mAbs are able to block acquisition of TGN1412 reactivity, illustrating the need for HLA recognition by the TCR of the cells acquiring this reactivity. High-density preculture of PBMC was performed as given in Figure 1B. To some high-density precultures, mAbs to HLA class I or HLA class II were added at 10µg/ml.

This finding very strongly suggests that interaction of the TCR with HLA molecules in the densely packed lymphoid organs is a prerequisite for the strong reactivity to TGN1412 as experienced by the volunteers in the London trial, and that this situation is mimicked by high-density culture in vitro, thus restoring the reactivity of circulating T-cells, which have lost cell-cell contact, to that of the T-cells in lymphoid organs. It also explains why the response to OKT3 is observed not only in precultured but also in fresh PBMC: Since in contrast to TGN1412, OKT3 addresses the TCR itself, there is no need for "priming" this receptor by interactions with HLA-molecules. The differential ability of HLA class I and - class II specific mAb to block the acquisition of reactivity to TGN1412 is explained by the fact that more than 90% of available HLA molecules in PBMC cultures are of class I.

### Example 7: Testing the ability of corticosteroids to control TGN1412-mediated cytokine release

The OKT3-induced cytokine storm is routinely manged by prevention or intervention with high-dose corticosteroids (Goldman et al., 1989). Until now, it was impossible to test the sensitivity of TGN1412-induced cytokine release to corticosteroids because no assay system existed. We therefore used the new method to compare the sensitivity of cytokine release in fresh and precultured PBMC to dexamethasone ("Dex", Sigma-Aldrich).

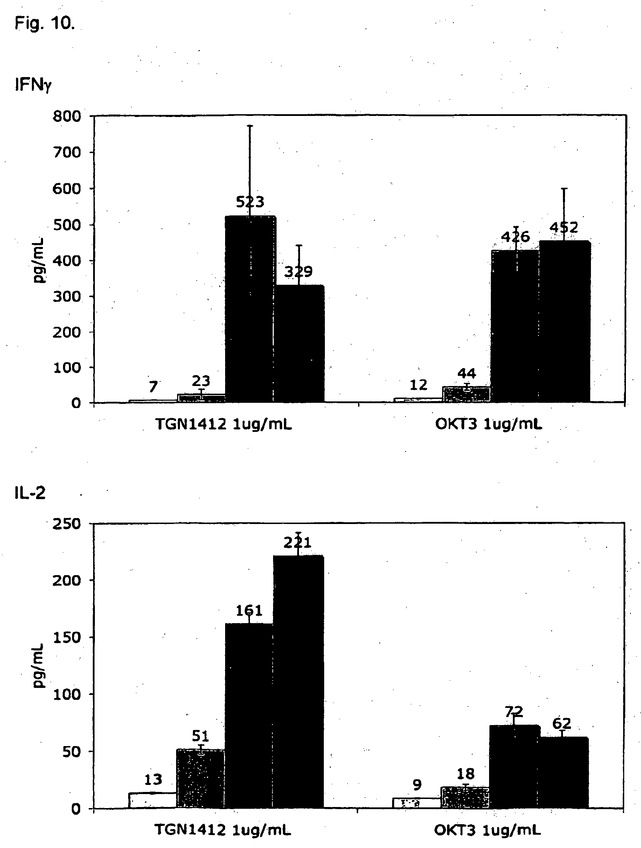

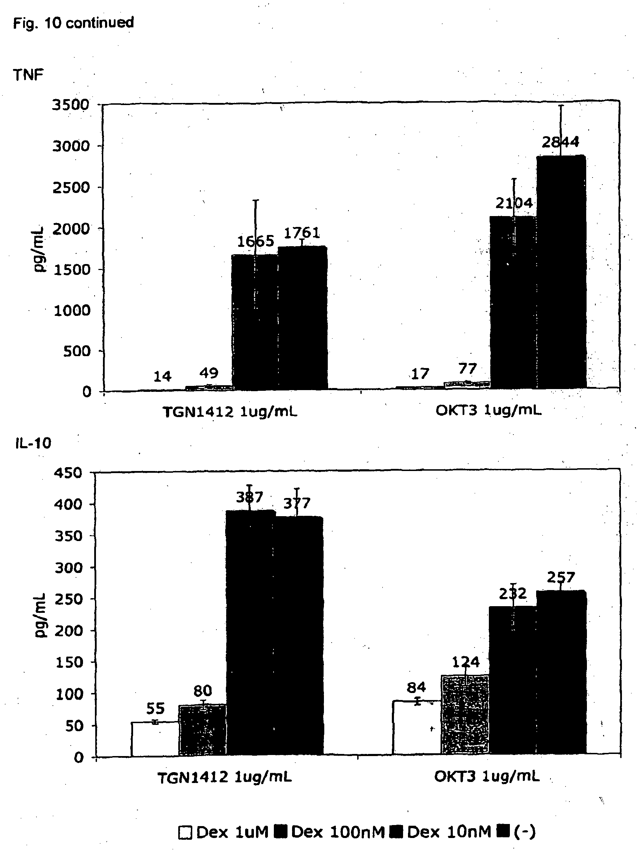

Figure 10 shows that TGN1412-induced cytokine release is sensititve to corticosteroids. High-density precultured cells were prepared and stimulated by mAb as described in Figure 1B. Where indicated, dexamethasone was included at the final concentrations given, and cytokines were measured after 24 hours of culture.

Cytokine release induced by both OKT3 and TGN1412 is fully suppressed by the highest dose employed (1µM), and is still almost completely suppressed at a tenfold lower dose (100nM). This strongly suggests that TGN1412 induced cytokine release can be controlled by appropriate corticosteroid medication as is clinically used for OKT3-treated patients.
Abramowicz, D. et al., Transplantation 47, 606-608 (1989).
Dennehy, K. M. et al., J Immunol 178, 1363-1371 (2007).
Dietz, A. B. et al., Transfusion 46, 2083-2089 (2006).
Duff, G. W. C.. Expert Scientific Group on Phase One Clinical Trials Final Report (Norwich, UK, Stationary Office 2006).
Gogishvili, T. et al., PLoS ONE 4, e4643 (2009).
Goldman, M. et al., Lancet 2, 802-803 (1989).
Hunig, T., Adv Immunol 95, 111-148 (2007).
Nguyen, D. H. et al., Proc Natl Acad Sci U S A 103, 7765-7770 (2006).
Schraven, B. et al., Immunity 28, 591-595 (2008).
Stefanova, I. et al., Nature 420, 429-434 (2002).
Suntharalingam, G. et al., N Engl J Med 355, 1018-1028 (2006).

## Claims

1. Method for testing a prospective or known immunomodulatory drug for T-cell activation, comprising
(a) the step of contacting in-vitro a peripheral blood mononuclear cell (PBMC) culture with a predetermined amount of the prospective or known immunomodulatory drug and
(b) observing the PBMC culture for release of at least one cytokine from the PBMCs or for cell proliferation upon contact with the prospective or known immunomodulatory drug,
wherein the PBMC culture of step (a) is a precultured PBMC culture, wherein the cell density of the PBMC preculture during and/or after the preculture step is at least 2*10⁶/ml or at least 4*10⁵/cm² such that cell-cell contact of the PBMCs is enabled, wherein the PBMC preculture is cultured for at least 24h, and wherein the PBMC preculture is cultured in the absence of immunomodulatory drugs, and prior to the contact with the prospective or known immunomodulatory drug to be tested.

2. The method according to claim 1, wherein the preculturing step is carried out by storing the PBMC culture for at least 36h, preferably at least 45 h, at 35°C to 40°C.

3. The method according to claim 1 or 2, wherein the immunomodulatory drug is an immunostimulating drug.

4. The method according to claim 3, wherein the immunostimulating drug is an antibody.

5. The method according to claim 4, wherein the antibody is a monoclonal antibody.

6. The method according to claim 5, wherein the monoclonal antibody is a human CD28 specific superagonistic monoclonal antibody.

7. Method according to any of the claims 1 to 6, wherein the cytokine observed is selected from the group consisting of TNF, IFN-gamma, IL-1-beta, IL-1, IL-2, IL-3, IL-4, IL-5, IL-6, IL-7, IL-8, IL-9, IL-10, IL12p70, IL-13, IL-14, IL-15, IL-16, IL-17, IL-21, IL-35, LT and combinations thereof.

8. Method according to any of the claims 1 to 7, wherein the precultured PBMC culture is additionally contacted with a prospective drug for attenuating the release of at least one cytokine, at the same time as contacting of the PBMC culture with the known immunomodulatory, in particular stimulating, drug or subsequently after a predetermined period of time, or a predetermined period of time there before, wherein the cytokine release is further observed.

9. Method according to claim 8, wherein the predetermined period of time can be in the range of 10 s to 12 h, preferably in the range of 10 s to 1 h.

10. Method according to any of the claims 8 or 9, wherein the prospective drug for attenuating the cytokine release is a corticosteroid.

## Patentansprüche

1. Verfahren zum Testen eines potentiellen oder bekannten immunmodulatorischen Medikaments zur T-Zellaktivierung, umfassend
(a) den Schritt des *in-vitro*-Inkontaktbringens einer Kultur von mononukleären Zellen des peripheren Bluts (PBMC) mit einer vorbestimmten Menge des potentiellen oder bekannten immunmodulatorischen Medikaments und
(b) die Überwachung der PBMC-Kultur auf die Freisetzung von mindestens einem Cytokin aus den PBMCs oder auf die Zellproliferation nach dem Kontakt mit dem potentiellen oder bekannten immunmodulatorischen Medikament,
wobei die PBMC-Kultur vom Schritt (a) eine vorgezüchtete PBMC-Kultur ist, wobei die Zelldichte einer PBMC-Vorkultur während und/oder nach dem Vorkulturschritt mindestens 2*10⁶/ml oder mindestens 4*10⁵/cm² ist, so dass ein Zell-Zell-Kontakt der PBMCs ermöglicht wird, wobei die PBMC-Vorkultur mindestens 24 Stunden gezüchtet wird und wobei die PBMC-Vorkultur in Abwesenheit von immunmodulatorischen Medikamenten gezüchtet wird und vor dem Kontakt mit dem zu testenden potentiellen oder bekannten immunmodulatorischen Medikament.

2. Verfahren nach Anspruch 1, wobei der Vorkultivierungsschritt durch Lagerung der PBMC-Kultur für mindestens 36 Stunden und bevorzugt mindestens 45 Stunden bei 35°C bis 40°C erfolgt.

3. Verfahren nach Anspruch 1 oder 2, wobei das immunmodulatorische Medikament ein immunstimulierendes Medikament ist.

4. Verfahren nach Anspruch 3, wobei das immunstimulierende Medikament ein Antikörper ist.

5. Verfahren nach Anspruch 4, wobei der Antikörper ein monoclonaler Antikörper ist.

6. Verfahren nach Anspruch 5, wobei der monoclonale Antikörper ein menschlicher CD28-spezifischer superagonistischer monoclonaler Antikörper ist.

7. Verfahren nach einem der Ansprüche 1 bis 6, wobei das überwachte Cytokin ausgewählt ist aus der Gruppe bestehend aus TNF, IFN-gamma, IL-1-beta, IL-1, IL-2, IL-3, IL-4, IL-5, IL-6, IL-7, IL-8, IL-9, IL-10, IL-12p70, IL-13, IL-14, IL-15, IL-16, IL-17, IL-21, IL-35, LT und Kombinationen davon.

8. Verfahren nach einem der Ansprüche 1 bis 7, wobei die vorkultivierte PBMC-Kultur zusätzlich mit einem potentiellen Medikament zur Attenuierung der Freisetzung mindestens eines Cytokins kontaktiert wird, zu der gleichen Zeit, zu der die PBMC-Kultur mit dem bekannten immunmodulatorischen, im besonderen stimulierenden, Medikament kontaktiert wird oder im Anschluss an eine vorbestimmte Zeitspanne oder zu einer vorbestimmten Zeitspanne davor, wobei die Cytokinfreisetzung weiter überwacht wird.

9. Verfahren nach Anspruch 8, wobei die vorbestimmte Zeitspanne im Bereich von 10 Sekunden bis 12 Stunden, vorzugsweise im Bereich von 10 Sekunden bis 1 Stunde sein kann.

10. Verfahren nach Anspruch 8 oder 9, wobei das potentielle Medikament zur Attenuierung der Cytokinfreisetzung ein Corticosteroid ist.

## Revendications

1. Procédé de test d'un médicament immunomodulateur connu ou prospectif pour sa capacité à activer les cellules T, comprenant :
(a) l'étape de mise en contact in vitro d'une culture de cellules sanguines mononucléaires périphériques (PBMC) avec une quantité prédéterminée du médicament immunomodulateur connu ou prospectif et
(b) d'observation de la culture de PBMC en termes de libération d'au moins une cytokine par les PBMCs ou de prolifération cellulaire après contact avec le médicament immunomodulateur connu ou prospectif,
dans lequel la mise en culture de l'étape (a) est une préculture de PBMC, dans laquelle la densité cellulaire de la préculture de PBMC pendant et/ou après l'étape de préculture est d'au moins 2*10⁶/ml ou d'au moins 4*10⁵/cm² de telle sorte qu'un contact cellule-cellule des PBMC est permis, dans lequel la préculture des PBMC est cultivée pendant au moins 24h, et dans lequel la préculture des PBMC est cultivée en l'absence de médicaments immunomodulateurs, et avant contact avec le médicament immunomodulateur à tester.

2. Procédé selon la revendication 1, dans lequel l'étape de préculture est mise en oeuvre par maintien de la culture de PBMC pendant au moins 36 h, préférentiellement au moins 45 h, à 35 à 40°C.

3. Procédé selon l'une quelconque des revendications 1 à 2, dans lequel le médicament immunomodulateur est un médicament immunostimulateur.

4. Procédé selon la revendication 3, dans lequel le médicament immunomodulateur est un anticorps.

5. Procédé selon la revendication 4, dans lequel l'anticorps est un anticorps monoclonal.

6. Procédé selon la revendication 5, dans lequel l'anticorps monoclonal est un anticorps monoclonal superagoniste humain spécifique des CD28.

7. Procédé selon l'une quelconque des revendications 1 à 6, dans lequel la cytokine observée est choisie dans le groupe constitué par le TNF, l'IFN-gamma, l'IL-1-bêta, l'IL-1, l'IL-2, l'IL-3, l'IL-4, l'IL-5, l'IL-6, l'IL-7, l'IL-8, l'IL-9, l'IL-10, l'IL-12p70, l'IL-13, l'IL-14, l'IL-15, l'IL-16, l'IL-17, l'IL-21, l'IL-35, le LT et leurs combinaisons.

8. Procédé selon l'une quelconque des revendications 1 à 7, dans lequel la culture de PBMC précultivées est en outre mise en contact avec un médicament prospectif pour atténuer la libération d'au moins une cytokine, en même temps que la mise en contact de la culture de PBMC avec le médicament immunomodulateur, en particulier stimulateur, connu ou ultérieurement après une période de temps prédéterminée, ou une période de temps prédéterminée antérieure, dans lequel la libération des cytokines est en outre observée.

9. Procédé selon la revendication 8, dans lequel la période de temps prédéterminée peut être dans la plage de 10 s à 12 h, de préférence dans la plage de 10 s à 1 h.

10. Procédé selon l'une quelconque des revendications 8 ou 9, dans lequel le médicament prospectif pour atténuer la libération des cytokines est un corticostéroïde.
